# EUROPEAN PATENT APPLICATION

(11) **EP 4 306 065 A1**
(43) Date of publication of application: **17.01.2024**
(21) Application number: 23181795.8
(22) Date of filing: 27.06.2023
(51) Int. Cl.: A61B 17/42, A61B 17/00

(54) **DEVICES AND SYSTEMS FACILITATING ACCESS TO THE UTERUS**

(30) Priority: 27.06.2022 US 202217850371
(71) Applicant: Covidien LP, Mansfield, MA 02048 (US)
(72) Inventor: WOOD, Timothy J, Woburn, 01801 (US)
(74) Representative: Maschio & Soames IP Ltd

(57) **Abstract**

A surgical stimulation system includes a clip configured to releasably engage a portion of a shaft of a surgical instrument and a stimulation generator configured to generate mechanical vibration energy and transmit the mechanical vibration energy to the clip to thereby mechanically vibrate the shaft of the surgical instrument to facilitate insertion of the shaft of the surgical instrument through an opening in tissue. A surgical system includes the surgical stimulation system and the surgical instrument and a surgical method utilizes the surgical stimulation system to facilitate insertion of the surgical instrument through an opening in tissue.

## Description

### FIELD

The present disclosure relates to hysteroscopy and, more particularly, to devices, systems, and methods facilitating access to the uterus, e.g., for performing one or more surgical tasks with the uterus.

### BACKGROUND

Trans-vaginal hysteroscopy involves the passage of an instrument or instruments through the external orifice of the vagina, through the transition between the vaginal cavity and the cervix (referred to as the external cervical os), and, finally, through the transition between the cervix and the uterus (referred to as the internal cervical os) and into the uterus. Of these three orifices, the internal cervical os is the smallest entry point. Depending on a patient's pain tolerance threshold and anatomical dimensions, the internal cervical os cannot be dilated beyond certain limits without the patients experiencing more than tolerable levels of discomfort, such as levels of discomfort for which anesthesia is needed.

### SUMMARY

As used herein, the term "distal" refers to the portion that is being described which is further from a user, while the term "proximal" refers to the portion that is being described which is closer to a user. The terms "substantially," "about," and the like as utilized herein are intended to encompass variations such as, for example, tolerances, measurement variations, design variations, environmental variations, etc., and encompass variations up to and including 10% (or greater if industry standards dictate). Further, to the extent consistent, any or all of the aspects detailed herein may be used in conjunction with any or all of the other aspects detailed herein.

Provided in accordance with aspects of the present disclosure is a surgical stimulation system including a sleeve and a stimulation generator. The sleeve is configured for releasable positioning about a portion of a shaft of a surgical instrument and includes a sleeve body and a plurality of electrodes disposed on or within the sleeve body. The stimulation generator is electrically coupled to the plurality of electrodes and configured to generate electrical stimulation energy and transmit the electrical stimulation energy to the plurality of electrodes for stimulating tissue in contact with the plurality of electrodes.

In an aspect of the present disclosure, the stimulation generator and the plurality of electrodes are configured to transmit Transcutaneous Electrical Nerve Stimulation (TENS) energy to tissue in contact with the plurality of electrodes.

In another aspect of the present disclosure, the stimulation generator and the plurality of electrodes are configured to transmit pulses of electrical energy to tissue in contact with the plurality of electrodes.

In another aspect of the present disclosure, the sleeve body is electrically insulative.

In still another aspect of the present disclosure, the stimulation generator is disposed within a console coupled to the sleeve via a cable.

In yet another aspect of the present disclosure, the system further includes a power source disposed within the console and configured to power the stimulation generator.

A surgical system provided in accordance with aspects of the present disclosure includes a surgical instrument including a shaft configured for insertion through an opening in tissue, and a surgical stimulation system. The surgical stimulation system includes a sleeve and a stimulation generator. The sleeve is configured for releasable positioning about a portion of the shaft of the surgical instrument and includes a sleeve body and a plurality of electrodes disposed on or within the sleeve body. The stimulation generator is electrically coupled to the plurality of electrodes and is configured to generate electrical stimulation energy and transmit the electrical stimulation energy to the plurality of electrodes for stimulating tissue in contact with the plurality of electrodes.

In an aspect of the present disclosure, the surgical instrument is a hysteroscope.

In another aspect of the present disclosure, the surgical instrument is configured to treat tissue within the uterus.

In another aspect of the present disclosure, the stimulation generator and the plurality of electrodes are configured to transmit Transcutaneous Electrical Nerve Stimulation (TENS) energy to tissue in contact with the plurality of electrodes.

In still another aspect of the present disclosure, the stimulation generator and the plurality of electrodes are configured to transmit pulses of electrical energy to tissue in contact with the plurality of electrodes.

In yet another aspect of the present disclosure, the sleeve body is electrically insulative.

In still yet another aspect of the present disclosure, the stimulation generator is disposed within a console coupled to the sleeve via a cable.

In another aspect of the present disclosure, the stimulation system further includes a power source disposed within the console and configured to power the stimulation generator.

A surgical method provided in accordance with aspects of the present disclosure includes attaching a sleeve to a shaft of a surgical instrument. The sleeve includes a sleeve body and a plurality of electrodes disposed on or within the sleeve body. The method further includes activating a stimulation generator to generate electrical stimulation energy and transmit the electrical stimulation energy to the plurality of electrodes, and inserting the shaft of the surgical instrument, with the sleeve disposed about the shaft, through an opening in tissue. The electrical stimulation energy stimulates tissue surrounding the opening in tissue to facilitate the insertion.

In an aspect of the present disclosure, the method further includes deactivating the stimulation generator once the shaft of the surgical instrument is inserted through the opening in tissue.

In another aspect of the present disclosure, the method further includes performing a surgical task with the surgical instrument after deactivating the stimulation generator.

In still another aspect of the present disclosure, inserting the shaft of the surgical instrument through the opening in tissue includes inserting the shaft of the surgical instrument through a cervix and into a uterus. In such aspects, the surgical instrument may be a hysteroscope and/or the surgical task includes visualizing the uterus.

In yet another aspect of the present disclosure, activating the stimulation generator to generate electrical stimulation energy and transmit the electrical stimulation energy to the plurality of electrodes includes transmitting Transcutaneous Electrical Nerve Stimulation (TENS) energy to tissue in contact with the plurality of electrodes.

Another surgical stimulation system provided in accordance with aspects of the present disclosure includes a clip and a stimulation generator. The clip is configured to releasably engage a portion of a shaft of a surgical instrument. The stimulation generator is configured to generate mechanical vibration energy and transmit the mechanical vibration energy to the clip to thereby mechanically vibrate the shaft of the surgical instrument to facilitate insertion of the shaft of the surgical instrument through an opening in tissue.

In an aspect of the present disclosure, the clip includes first and second arms. Each of the first and second arms includes a clamping portion. The first and second arms are movable relative to one another to clamp the portion of the shaft between the clamping portions of the first and second arms. In such aspects, the clamping portions of the first and second arms may be biased towards one another, thereby providing bias to maintain the clip in engagement about the portion of the shaft.

In another aspect of the present disclosure, a sterile barrier is configured for positioning about the portion of the shaft of the surgical instrument. In such aspects, the clip is configured to engage the portion of the shaft of the surgical instrument and transmit the mechanical vibration energy thereto through the sterile barrier.

In still another aspect of the present disclosure, the surgical stimulation system further includes a portable power source configured to power the stimulation generator.

In yet another aspect of the present disclosure, the stimulation generator is disposed on or within the clip.

In still yet another aspect of the present disclosure, the stimulation generator is configured to operate in a range of about 10 Hz to about 1000 Hz.

Another surgical system provided in accordance with aspects of the present disclosure includes a surgical instrument including a shaft configured for insertion through an opening in tissue, and a surgical stimulation system. The surgical stimulation system includes a clip configured to releasably engage a portion of the shaft of the surgical instrument, and a stimulation generator configured to generate mechanical vibration energy and transmit the mechanical vibration energy to the clip to thereby mechanically vibrate the shaft of the surgical instrument to facilitate insertion of the shaft of the surgical instrument through the opening in tissue.

In an aspect of the present disclosure, the surgical instrument is a hysteroscope.

In another aspect of the present disclosure, the surgical instrument is configured to treat tissue within the uterus.

In another aspect of the present disclosure, the clip includes first and second arms configured to clamp the portion of the shaft between clamping portions of the first and second arms. The clamping portions of the first and second arms are biased towards one another to thereby provide bias to maintain the clip in engagement about the portion of the shaft.

In yet another aspect of the present disclosure, the system further includes a sterile barrier configured for positioning about the portion of the shaft of the surgical instrument. In such aspects, the clip is configured to engage the portion of the shaft of the surgical instrument and transmit the mechanical vibration energy thereto through the sterile barrier.

In still another aspect of the present disclosure, the system further includes a portable power source configured to power the stimulation generator.

In still yet another aspect of the present disclosure, the stimulation generator is disposed on or within the clip.

In another aspect of the present disclosure, the stimulation generator is configured to operate in a range of about 10 Hz to about 1000 Hz.

Another surgical method provided in accordance with the present disclosure includes attaching a clip to a portion shaft of a surgical instrument, activating a stimulation generator to generate mechanical vibration energy and transmit the mechanical vibration energy to the clip for mechanically vibrating the shaft of the surgical instrument, and inserting the shaft of the surgical instrument through an opening in tissue. The mechanical vibration of the shaft of the surgical instrument stimulates tissue surrounding the opening in tissue to facilitate the insertion.

In an aspect of the present disclosure, the method further includes removing the clip from the portion of the shaft of the surgical instrument. In such aspects, the method may include performing a surgical task after removing the clip.

In another aspect of the present disclosure, the method further includes positioning a sterile barrier about the portion of the shaft prior to attaching the clip such that the clip is engaged to the portion of the shaft with the sterile barrier between the clip and the portion of the shaft.

In yet another aspect of the present disclosure, inserting the shaft of the surgical instrument through the opening in tissue includes inserting the shaft of the surgical instrument through a cervix and into a uterus.

### BRIEF DESCRIPTION OF DRAWINGS

The above and other aspects and features of the present disclosure will become more apparent in view of the following detailed description when taken in conjunction with the accompanying drawings wherein like reference numerals identify similar or identical elements.
FIG. 1A is a perspective view of a hysteroscope configured for use in accordance with the present disclosure;
FIG. 1B is a transverse, cross-sectional view taken across section line "1B-1B" of FIG. 1A
FIG. 2A is a perspective view of another hysteroscope configured for use in accordance with the present disclosure;
FIG. 2B is an exploded, side view of the hysteroscope of FIG. 2A with the sheath removed from the body;
FIG. 3 is a perspective view of a tissue resecting instrument configured for use in accordance with the present disclosure;
FIG. 4 is a perspective view of the hysteroscope of FIG. 1A including a stimulation system in accordance with the present disclosure operably coupled to the hysteroscope;
FIG. 5 is an exploded, perspective view of the hysteroscope of FIG. 1A, the stimulation system of FIG. 4, and a sterile barrier configured for positioning between the hysteroscope and the stimulation system to maintain sterility of the hysteroscope;
FIG. 6 is a perspective view of the hysteroscope of FIG. 1A including another stimulation system in accordance with the present disclosure operably coupled to the hysteroscope; and
FIG. 7 is a cross-sectional illustration of a distal end portion of the hysteroscope of FIG. 1A.

### DETAILED DESCRIPTION

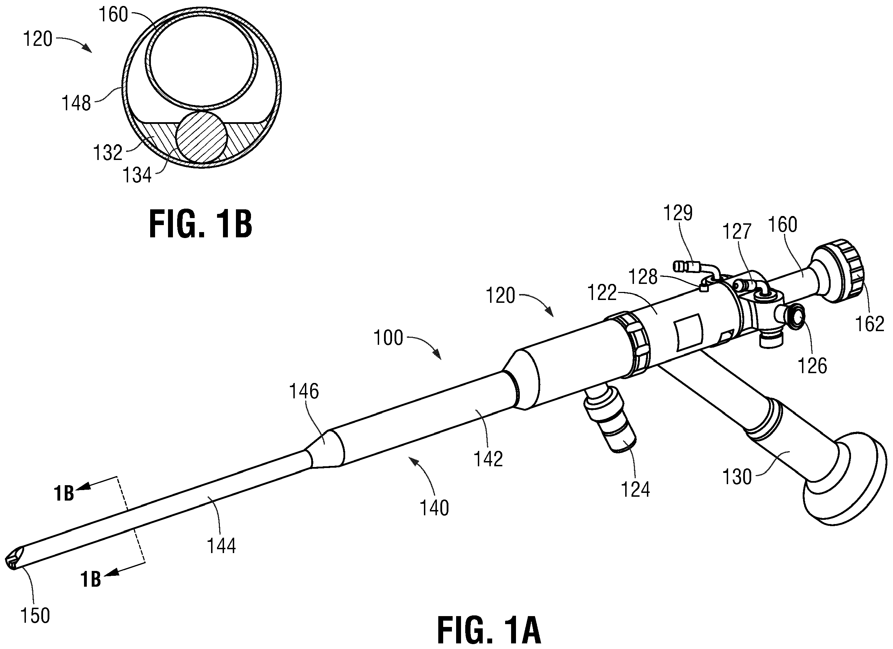

Referring to FIGS. 1A and 1B, a hysteroscope configured for use in accordance with the present disclosure is shown generally identified by reference numeral 100 including a proximal handle 120, a distal shaft 140, and a removable outflow and/or instrument working channel 160.

Proximal handle 120 of hysteroscope 100 includes a housing 122, a light post 124, inflow and outflow ports 126, 128, respectively, inflow and outflow valves 127, 129, respectively, and an arm 130. Housing 122 defines a generally cylindrical-shaped configuration extending along a longitudinal axis defined by hysteroscope 100. Light post 124 extends from housing 122 and is configured to connect to a light source, e.g., to illuminate a distal end of distal shaft 140 via a plurality of fiber optic strands 132 (FIG. 1A) coupled to light post 124 and extending through distal shaft 140 to the distal end portion of distal shaft 140. Inflow and outflow ports 126, 128 enable connection of a fluid supply and a fluid collection container, respectively, to hysteroscope 100 to enable the inflow and outflow of fluid through distal shaft 140 to/from the internal surgical site, e.g., the uterus. Inflow and outflow valves 127, 129 enable the selective control of the fluid resistance through inflow and outflow ports 126, 128, respectively, thereby enabling control of fluid flow rate into and out of the internal surgical site. Arm 130 is configured to connect to an imaging device, e.g., a camera, to capture images received via optics 134 (FIG. 1A) extending through distal shaft 140 to the distal end portion of distal shaft 140 and, thus, enable display of a video image of the internal surgical site as captured by optics 134.

Distal shaft 140 of hysteroscope 100 extends distally from proximal handle 120 along the longitudinal axis of hysteroscope 100 and includes including a proximal portion 142 having a first diameter, a distal portion 144 having a second, smaller diameter, and a transition portion 146 disposed between proximal and distal portions 142, 144, respectively, and tapering from the first diameter to the second diameter to provide a transition between proximal and distal portions 142, 144, respectively. Distal shaft 140 includes, as noted above, the plurality of fiber optic strands 132 and optics 134 extending therethrough. Distal shaft 140 further includes a generally D-shaped inflow channel 148 extending therethrough. Inflow channel 148 fluidly communicates with inflow port 126. The plurality of fiber optic strands 132 and optics 134 extend through distal shaft 140 externally of inflow channel 148. Working channel 160 extends through inflow channel 148 and is removable therefrom, although in aspects, working channel 160 is integral with distal shaft 140. Working channel 160 functions as an outflow and/or instrument channel that fluidly communicates with outflow port 128. In aspects, the channels 148, 160 may be reversed, e.g., wherein channel 148 is utilized for outflow and channel 160 is utilized for inflow. In aspects, working channel 160 includes a proximal seal assembly 162 configured to establish a seal about an instrument inserted through working channel 160.

The distal tip 150 of distal shaft 140 includes a partially-slanted configuration whereby the plurality of fiber optic strands 132 and optics 134 end at a perpendicular distal surface while the inflow channel 148 defines a slanted distal surface that is angled proximally from the distal surface of the plurality of fiber optic strands 132 and optics 134.

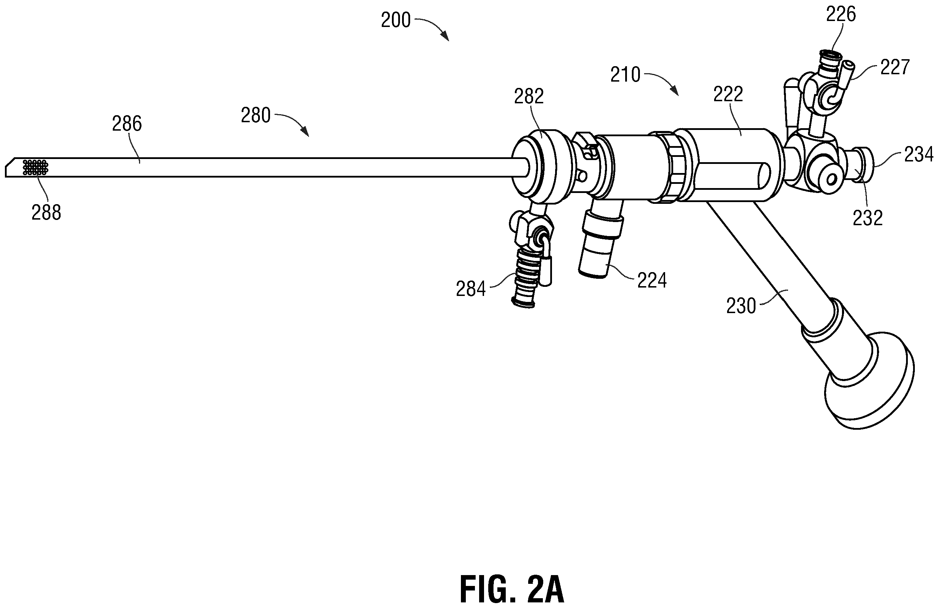

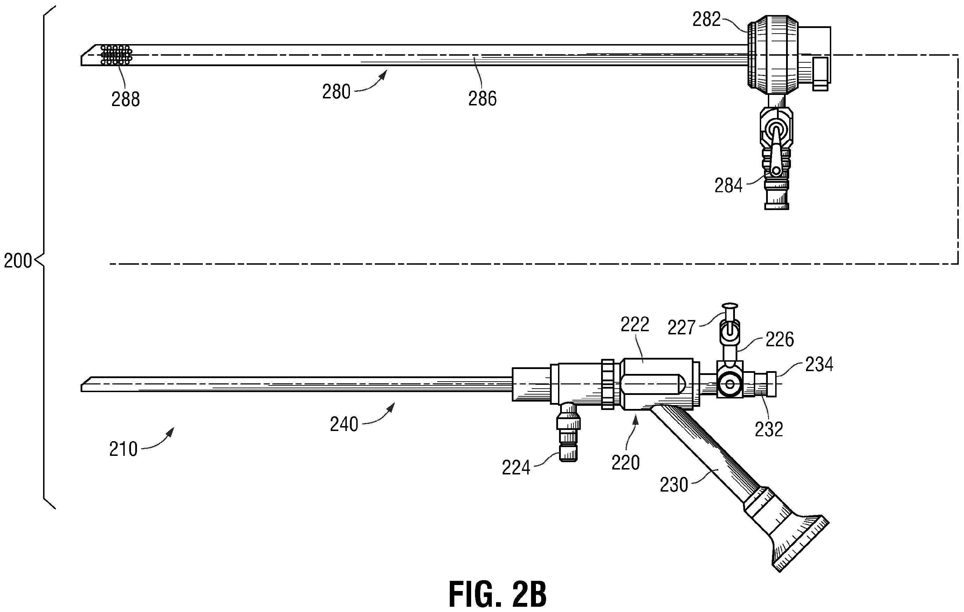

Referring to FIGS. 2A and 2B, another hysteroscope configured for use in accordance with the present disclosure is shown generally identified by reference numeral 200 including a body 210 and a sheath 280 configured to releasably connect to body 210. Body 210 includes a proximal handle 220 and a distal shaft 240 extending distally from proximal handle 220.

Proximal handle 220 of body 210 of hysteroscope 200 includes a housing 222, a light post 224, a first port 226, a first valve 227, and an arm 230, each of which may be configured similarly as detailed above with respect to the corresponding components of hysteroscope 100 (FIG. 1A). Proximal handle 220 further includes an insertion port 232 disposed at a proximal end thereof configured to enable insertion of a surgical instrument through proximal handle 220 and distal shaft 240 and into an internal surgical site. In aspects, insertion port 232 includes a proximal seal assembly 234 configured to establish a seal about an instrument inserted through insertion port 232.

Distal shaft 240 of body 210 of hysteroscope 200 extends distally from proximal handle 220 along the longitudinal axis of hysteroscope 200. Distal shaft 240 may be configured similar to distal shaft 140 of hysteroscope 100 (FIGS. 1A and 1B) except that working channel 160 (FIGS. 1A and 1B) is omitted. Rather, to provide outflow (or inflow, in configurations where inflow and outflow are reversed), hysteroscope 200 includes sheath 280. Sheath 280 includes a proximal hub 282 configured to enable releasable engagement of sheath 280 about distal shaft 240 of body 210 of hysteroscope 200, an outflow port 284 communicating with the interior of sheath 280 to enable the outflow of fluid through sheath 280 (or, in the alternative, the inflow of fluid through sheath 280), and an elongated sleeve 286 extending distally from proximal hub 282 and configured to substantially surround distal shaft 240 of body 210 of hysteroscope 200 to define an annular outflow (or inflow) channel therebetween. In aspects, elongated sleeve 286 defines a plurality of apertures 288 transversely therethrough towards the distal end thereof to facilitate outflow (or inflow) through the annular channel.

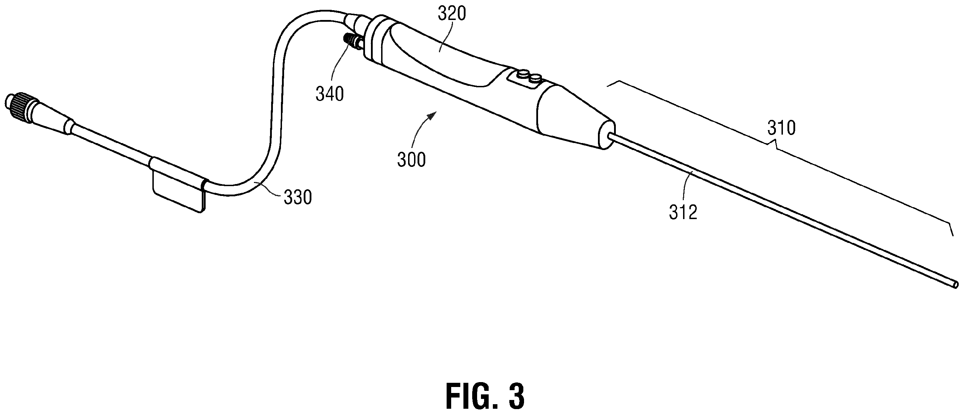

Turning to FIG. 3, a tissue resecting instrument 300 configured for use in accordance with the present disclosure and configured to resect tissue includes an end effector assembly 310 and a handpiece assembly 320. Tissue resecting instrument 300 is adapted to connect to a control unit (not shown) via a cable 330 to provide power and control functionality to tissue resecting instrument 300, although tissue resecting instrument 300 may alternatively or additionally include controls associated with handpiece assembly 320 and/or a power source, e.g., battery, disposed within handpiece assembly 320. Tissue resecting instrument 300 is further adapted to connect to a fluid management system (not shown) via outflow tubing connected to outflow port 340 for applying suction to remove fluid, tissue, and debris from a surgical site via tissue resecting instrument 300. The control unit and fluid management system may be integral with one another, coupled to one another, or separate from one another.

End effector assembly 310 may be configured to releasable engage handpiece assembly 320 or may be integrally formed with handpiece assembly 320. Tissue resecting instrument 300 may include a motor (not shown) disposed within handpiece assembly 320 and configured to drive translation, rotation, oscillation, and/or other suitable movement of an inner shaft (not shown) of end effector assembly 310 relative to an outer shaft 312 of end effector assembly 310 (or vice versa) to facilitate cutting tissue. The applied suction through tissue resecting instrument 300 functions to withdraw the cut tissue, along with fluids and/or debris, from the surgical site through tissue resecting instrument 300.

Although hysteroscopes 100, 200 (FIGS. 1A and 2A, respectively) and tissue resecting instrument 300 (FIG. 3) are shown and described herein, it is contemplated that other suitable surgical instruments may be configured for use in accordance with the present disclosure such as, for example, and without limitation, endoscopes, surgical cameras, ultrasound probes, ablation probes, biopsy devices, uterine manipulators, cervical dilators, curettes, and forceps. Likewise, although the present disclosure is detailed with respect to accessing the uterus via the cervix, the aspects and features of the present disclosure apply equally to accessing other internal surgical sites via naturally-occurring and/or surgically-created openings.

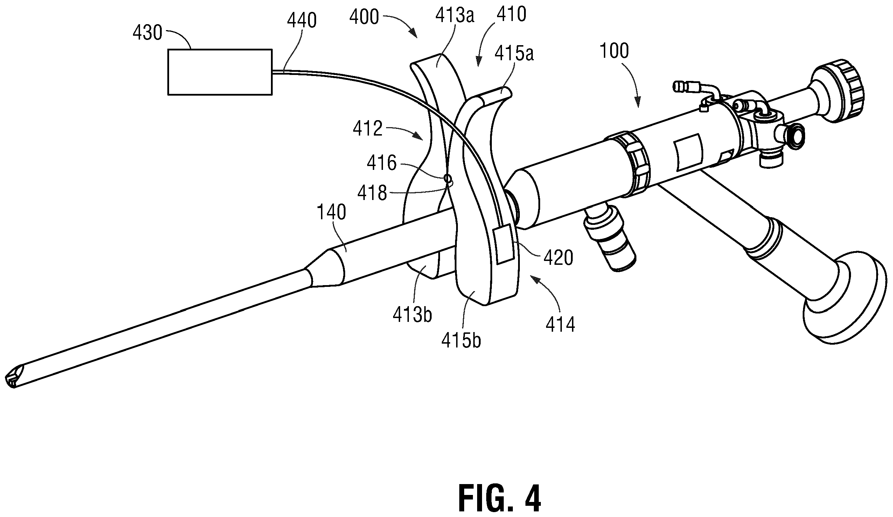

Turning to FIG. 4, a stimulation system provided in accordance with the present disclosure is shown generally identified by reference numeral 400 including a clip 410, a stimulation generator 420, and a power source 430. Stimulation generator 420 is mounted on or within clip 410, although stimulation generator 420 may alternatively be detached from and operably coupled to clip 410. Power source 430 may be connected to stimulation generator 420 via a cable 440, or, in the alternative, may be mounted on or within clip 410 together with or separately from stimulation generator 420.

Clip 410 is configured to releasably clamp onto a shaft (or other elongated portion) of a surgical instrument such as, for example, distal shaft 140 of hysteroscope 100. Clip 410 includes first and second arms 412, 414 each including a handle portion 413a, 414a and a clamping portion 413b, 414b, respectively. Arms 412, 414 are coupled to one another about a pivot 416 disposed between respective handle portions 413a, 415a and clamping portions 413b, 415b thereof. A biasing member 418, e.g., a torsion spring disposed about pivot 416, may be provided to bias handle portions 413a, 415a apart from one another, thereby biasing clamping portions 413b, 415b towards one another. Thus, in order to position clip 410 about distal shaft 140 of hysteroscope 100, handle portions 413a, 415a are squeezed towards one another and against the bias of biasing member 418 sufficiently so as to enable passage of clamping portions 413b, 415b on either side of distal shaft 140. Once this position has been achieved, handle portions 413a, 415a may be released such that clamping portions 413b, 415b engage distal shaft 140 on either side thereof and are maintained in engagement clamping distal shaft 140 under the bias of biasing member 418. Other suitable releasably engagable clip mechanisms are also contemplated.

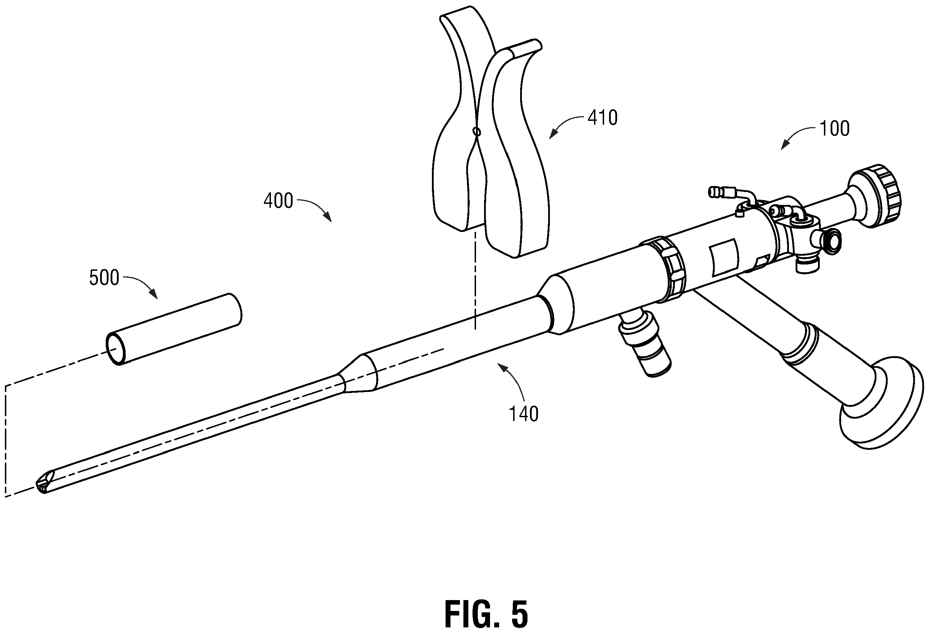

With momentary reference to FIG. 5, in aspects, rather than clip 410 directly engaging distal shaft 140 of hysteroscope 100, a sterile barrier 500, e.g., a sterile sheath, may be disposed on at least a portion of distal shaft 140 at least in the vicinity of where clip 410 is to be positioned. Thereafter, clip 410 may be engaged about distal shaft 140 as detailed above with sterile barrier 500 therebetween. Thus, the sterility of hysteroscope 100 can be maintained and direct contact between clip 410 and hysteroscope 100 can be avoided.

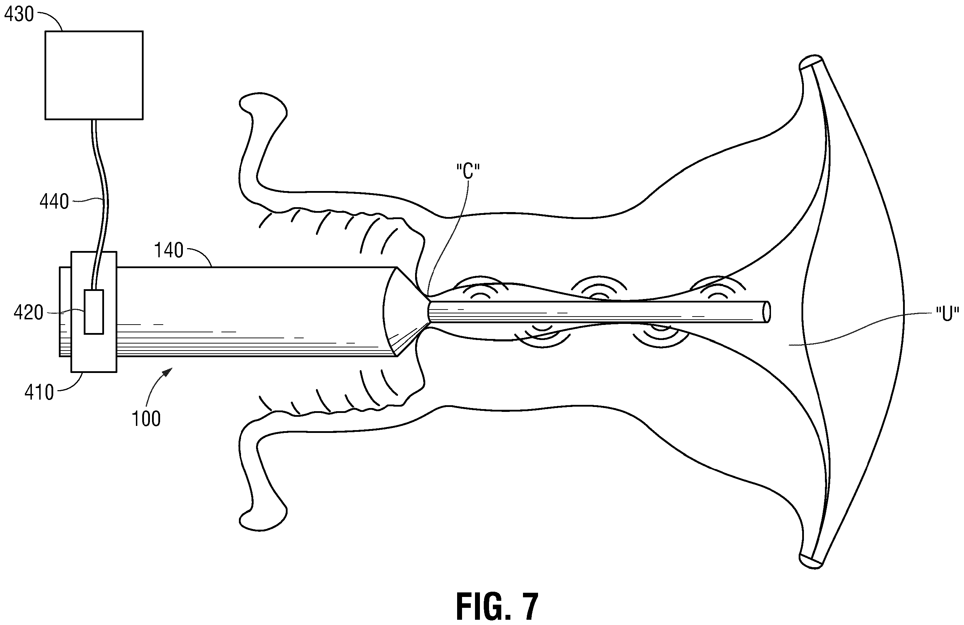

Referring back to FIG. 4, stimulation generator 420 may be a mechanical vibration generator such as, for example, a vibration motor, a transducer, a mechanical oscillator, etc., configured to generate mechanical vibration energy that is transmitted to distal shaft 140 via clip 410 when clip 410 is engaged about distal shaft 140. The vibration energy produced by stimulation generator 420 and transmitted to distal shaft 140 is configured to vibrate distal shaft 140 to stimulate tissue in contact therewith, thus facilitating insertion of distal shaft 140 through the cervix "C" and into the uterus "U" (see FIG. 7) and reducing discomfort during insertion. Stimulation generator 420 may be configured to operate, in aspects, within a range of about 10 Hz to about 1000 Hz; in other aspects, within a range of about 10 Hz to about 500 Hz; about 500 Hz; within a range of about 30Hz - 50Hz, or any other suitable frequency or frequency range.

Power source 430 may be a portable power source such as, for example, a battery pack including one or more batteries (rechargeable or disposable). Alternatively, power source 430 may be a surgical generator or a standard wall outlet. In such aspects, cable 440 includes a corresponding plug (not explicitly shown) configured to interface with power source 430.

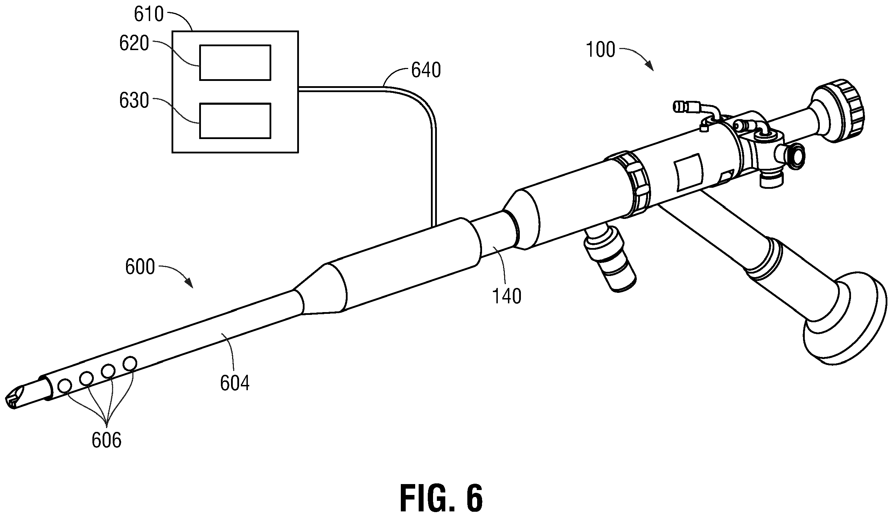

Turning to FIG. 6, another stimulation system provided in accordance with the present disclosure is shown generally identified by reference numeral 600 including a sleeve 602 and a console 610 including a stimulation generator 620 and a power source 630, although it is also contemplated that stimulation generator 620 and power source 630 be separate from one another.

Sleeve 602 is configured to releasably slide over at least a portion of a shaft (or other elongated portion) of a surgical instrument such as, for example, distal shaft 140 of hysteroscope 100. Sleeve 602 may define an enclosed annular configuration such that sleeve 602 is slid over an end of distal shaft 140, e.g., the distal end thereof, and into position. Alternatively, sleeve 602 may define a C-shaped or other suitable configuration to enable transverse engagement of sleeve 602 about distal shaft 140 at a desired location.

Sleeve 602 includes a sleeve body 604 having a plurality of electrodes 606 disposed thereon or therein configured to deliver electrical stimulation energy to tissue in contact with electrodes 606. Sleeve body 604 may be formed from an electrically-insulative material. The electrodes 606 may be configured to deliver to tissue: Transcutaneous Electrical Nerve Stimulation (TENS) energy, Electrical Muscle Stimulation (EMS) energy, RF energy, microwave energy, light energy, or any other suitable electrical energy to tissue to stimulate tissue, thus facilitating insertion of distal shaft 140 through the cervix "C" and into the uterus "U" (see FIG. 7) and reducing discomfort during insertion.

Sleeve 602 may be configured as a disposable component that is removed and discarded after a single use. Thus, a new, sterile sleeve 602 may be provided for each use. In such and other aspects, sleeve 602 may be configured to releasably connect to a cable 640 and/or cable 640 may be configured to releasably connect to stimulation generator 620, thus enabling repeated use of stimulation generator 620 and/or cable 640 while sleeve 602 is single-use. Other configurations of sleeve 602 such as reusable configurations are also contemplated.

Stimulation generator 620 is electrically coupled to electrodes 606 via cable 640 and configured to generate electrical stimulation energy for transmission to electrodes 606 for stimulating tissue. Stimulation generator 620 may be configured to provide continuous energy, pulsed energy, and/or any other suitable form of electrical stimulation energy to electrodes 606. Power source 630 is configured to power stimulation generator 620 and may be a portable power source such as, for example, a battery pack including one or more batteries (rechargeable or disposable). Alternatively, power source 630 may be a surgical generator or a standard wall outlet.

With reference to FIG. 7, hysteroscope 100 is shown wherein distal shaft 140 is inserted trans-vaginally through the cervix "C" and into the uterus "U." During insertion, stimulation generator 420 is activated such that clip 410 transmits vibration energy to distal shaft 140. Thus, as distal shaft 140 is moved into contact with tissue surrounding the cervix "C," the vibration energy transmitted along distal shaft 140 stimulates the tissue, thus facilitating insertion of distal shaft 140 through the cervix "C" and into the uterus "U" and reducing discomfort during insertion. Once distal shaft 140 is positioned as desired, stimulation generator 420 may be deactivated and clip 410 removed from about distal shaft 140 for the remainder of the surgical procedure.

Although FIG. 7 illustrates the use of hysteroscope 100 with stimulation system 400 (FIG. 4), stimulation system 600 (FIG. 6) is utilized in a similar manner. That is, referring also to FIG. 6, during insertion of distal shaft 140 trans-vaginally through the cervix "C" and into the uterus "U," stimulation generator 620 is activated such that electrodes 606 transmit electrical energy to tissue surrounding the cervix "C" to stimulate the tissue, thus facilitating insertion of distal shaft 140 through the cervix "C" and into the uterus "U" and reducing discomfort during insertion. Once distal shaft 140 is positioned as desired, stimulation generator 620 may be deactivated. Sleeve 602 may remain in position about distal shaft 140 throughout the procedure or may be removed after insertion is achieved.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects. Those skilled in the art will envision other modifications within the scope and spirit of the claims appended hereto.

The invention may be described by reference to the following paragraphs:-
1. A surgical stimulation system, comprising: a clip configured to releasably engage a portion of a shaft of a surgical instrument; and a stimulation generator configured to generate mechanical vibration energy and transmit the mechanical vibration energy to the clip to thereby mechanically vibrate the shaft of the surgical instrument to facilitate insertion of the shaft of the surgical instrument through an opening in tissue.
2. The surgical stimulation system according to paragraph 1, wherein the clip includes first and second arms, each of the first and second arms including a clamping portion, the first and second arms movable relative to one another to clamp the portion of the shaft between the clamping portions of the first and second arms.
3. The surgical stimulation system according to paragraph 2, wherein the clamping portions of the first and second arms are biased towards one another, thereby providing bias to maintain the clip in engagement about the portion of the shaft.
4. The surgical stimulation system according to paragraph 1, further comprising a sterile barrier configured for positioning about the portion of the shaft of the surgical instrument, and wherein the clip is configured to engage the portion of the shaft of the surgical instrument and transmit the mechanical vibration energy thereto through the sterile barrier.
5. The surgical stimulation system according to paragraph 1, further comprising a portable power source configured to power the stimulation generator.
6. The surgical stimulation system according to paragraph 1, wherein the stimulation generator is disposed on or within the clip.
7. The surgical stimulation system according to paragraph 1, wherein the stimulation generator is configured to operate in a range of about 10 Hz to about 1000 Hz.
8. A surgical system, comprising: a surgical instrument including a shaft configured for insertion through an opening in tissue; and a surgical stimulation system, including: a clip configured to releasably engage a portion of the shaft of the surgical instrument; and a stimulation generator configured to generate mechanical vibration energy and transmit the mechanical vibration energy to the clip to thereby mechanically vibrate the shaft of the surgical instrument to facilitate insertion of the shaft of the surgical instrument through the opening in tissue.
9. The surgical system according to paragraph 8, wherein the surgical instrument is a hysteroscope.
10. The surgical system according to paragraph 8, wherein the surgical instrument is configured to treat tissue within the uterus.
11. The surgical system according to paragraph 8, wherein the clip includes first and second arms configured to clamp the portion of the shaft between clamping portions of the first and second arms, the clamping portions of the first and second arms biased towards one another to thereby provide bias to maintain the clip in engagement about the portion of the shaft.
12. The surgical system according to paragraph 8, further comprising a sterile barrier configured for positioning about the portion of the shaft of the surgical instrument, and wherein the clip is configured to engage the portion of the shaft of the surgical instrument and transmit the mechanical vibration energy thereto through the sterile barrier.
13. The surgical system according to paragraph 8, further comprising a portable power source configured to power the stimulation generator.
14. The surgical system according to paragraph 8, wherein the stimulation generator is disposed on or within the clip.
15. The surgical system according to paragraph 8, wherein the stimulation generator is configured to operate in a range of about 10 Hz to about 1000 Hz.
16. A surgical method, comprising: attaching a clip to a portion of a shaft of a surgical instrument; activating a stimulation generator to generate mechanical vibration energy and transmit the mechanical vibration energy to the clip for mechanically vibrating the shaft of the surgical instrument; and inserting the shaft of the surgical instrument through an opening in tissue, wherein the mechanical vibration of the shaft of the surgical instrument stimulates tissue surrounding the opening in tissue to facilitate the insertion.
17. The surgical method according to paragraph 16, further comprising removing the clip from the portion of the shaft of the surgical instrument.
18. The surgical method according to paragraph 17, further comprising performing a surgical task after removing the clip.
19. The surgical method according to paragraph 16, further comprising positioning a sterile barrier about the portion of the shaft prior to attaching the clip such that the clip is engaged to the portion of the shaft with the sterile barrier between the clip and the portion of the shaft.
20. The surgical method according to paragraph 16, wherein inserting the shaft of the surgical instrument through the opening in tissue includes inserting the shaft of the surgical instrument through a cervix and into a uterus.

## Claims

1. A surgical stimulation system, comprising:
a clip configured to releasably engage a portion of a shaft of a surgical instrument; and
a stimulation generator configured to generate mechanical vibration energy and transmit the mechanical vibration energy to the clip to thereby mechanically vibrate the shaft of the surgical instrument to facilitate insertion of the shaft of the surgical instrument through an opening in tissue.

2. The surgical stimulation system according to claim 1, wherein the clip includes first and second arms, each of the first and second arms including a clamping portion, the first and second arms movable relative to one another to clamp the portion of the shaft between the clamping portions of the first and second arms.

3. The surgical stimulation system according to claim 2, wherein the clamping portions of the first and second arms are biased towards one another, thereby providing bias to maintain the clip in engagement about the portion of the shaft.

4. The surgical stimulation system according to any preceding claim, further comprising a sterile barrier configured for positioning about the portion of the shaft of the surgical instrument, and wherein the clip is configured to engage the portion of the shaft of the surgical instrument and transmit the mechanical vibration energy thereto through the sterile barrier.

5. The surgical stimulation system according to any preceding claim, further comprising a portable power source configured to power the stimulation generator.

6. The surgical stimulation system according to any preceding claim, wherein the stimulation generator is disposed on or within the clip.

7. The surgical stimulation system according to any preceding claim, wherein the stimulation generator is configured to operate in a range of about 10 Hz to about 1000 Hz.

8. A surgical system, comprising:
a surgical instrument including a shaft configured for insertion through an opening in tissue; and
a surgical stimulation system, including:
a clip configured to releasably engage a portion of the shaft of the surgical instrument; and
a stimulation generator configured to generate mechanical vibration energy and transmit the mechanical vibration energy to the clip to thereby mechanically vibrate the shaft of the surgical instrument to facilitate insertion of the shaft of the surgical instrument through the opening in tissue.

9. The surgical system according to claim 8, wherein the surgical instrument is a hysteroscope.

10. The surgical system according to claim 8 and claim 9, wherein the surgical instrument is configured to treat tissue within the uterus.

11. The surgical system according to any of claims 8 to 10, wherein the clip includes first and second arms configured to clamp the portion of the shaft between clamping portions of the first and second arms, the clamping portions of the first and second arms biased towards one another to thereby provide bias to maintain the clip in engagement about the portion of the shaft.

12. The surgical system according to any of claims 8 to 11, further comprising a sterile barrier configured for positioning about the portion of the shaft of the surgical instrument, and wherein the clip is configured to engage the portion of the shaft of the surgical instrument and transmit the mechanical vibration energy thereto through the sterile barrier.

13. The surgical system according to any of claims 8 to 12, further comprising a portable power source configured to power the stimulation generator.

14. The surgical system according to any of claims 8 to 13, wherein the stimulation generator is disposed on or within the clip.

15. The surgical system according to any of claims 8 to 14, wherein the stimulation generator is configured to operate in a range of about 10 Hz to about 1000 Hz.
